# EUROPEAN PATENT SPECIFICATION

(11) **EP 2 782 535 B1**
(45) Date of publication and mention of the grant of the patent: **19.10.2016**
(21) Application number: 12799403.6
(22) Date of filing: 21.11.2012
(51) Int. Cl.: A61F 13/00, A61L 27/60, A61L 29/16, A61L 31/16, A61F 2/07, A61B 17/322

(54) **MODIFIABLE MEDICAL GRAFTS AND RELATED APPARATUSES**
MEDIZINISCHE MODIFIZIERBARE TRANSPLANTATE SOWIE ENTSPRECHENDE VORRICHTUNGEN
GREFFONS MÉDICAUX MODIFIABLES ET APPAREILS ASSOCIÉS

(30) Priority: 24.11.2011 US 201161563600 P
(43) Date of publication of application: 01.10.2014
(73) Proprietor: Cook Biotech Incorporated, West Lafayette, IN 47906 (US); Muffin Incorporated, West Lafayette, IN 47906 (US); Cook Medical Technologies LLC, Bloomington, IN 47404 (US)
(72) Inventor: FEARNOT, Neal, E., West Lafayette, IN 47906 (US); HILES, Michael, C., West Lafayette, IN 47905 (US); METZ, Jeremy, Lafayette, IN 47905 (US); OBERMILLER, F., Joseph, West Lafayette, IN 47906 (US); KAUCHER, Andrew, J., Greenfield, IN 46140 (US); CHARLEBOIS, Steven, West Lafayette, IN 47906 (US)
(74) Representative: Williams Powell
(86) International application number: PCT/US2012/066263
(87) International publication number: WO 2013/078315

(56) References cited:
- GB-A- 2 451 785
- US-A- 6 001 123
- US-A1- 2007 123 977
- US-A1- 2008 107 750
- US-A1- 2010 155 282

## Description

### BACKGROUND

The present invention relates in certain of its aspects to medical devices for example involving medical graft materials that can be modified by the addition of cells or other substances, and to related kits and methods and preparation.

Medical grafts and implants have demonstrated significant promise to improve medical treatments for patients across a broad variety of conditions or injuries. One area of study has been that of implantable graft materials that contain additives such as bioactive substances or viable cells from the patient or from other sources. With regard to the harvest and re-introduction of cells or other biologic substances from the patient, termed autologous treatments, methods and systems are known for treating tissue samples from the patient to result in a cellular and/or other biological preparation that can be re-introduced to the patient. In related areas, cells or biologic substances obtained from donors other than the patient, sometimes embodied in stable cell lines or purified extracts, have also been known or proposed for introduction into the patient by themselves or with other implant materials.

In certain modes of use, cells or other substances to be introduced into the patient can be combined with a graft material to form an implantable graft. In respect of cellular grafts, sometimes these involve a culture period in which the number of cells is expanded after application to the graft material. Other modes of using cells do not involve such expansion. Rather, the cells are applied to the graft and implanted without a culture period.

Despite demonstrated promise, the clinical implementation of graft materials has been slow in many areas. Needs exists for more convenient and/or effective ways or materials for combining cells or other modifying substances with graft materials. In certain of its aspects, the present invention is addressed to these needs.

US 2007/123977 A1 discloses an implantable medical device having a three-dimensional structure with a coating including drugs. US 2010/155282 A1 discloses a medical graft apparatus comprising a graft container with a cavity for keeping the graft under a vacuum. US 2008/107750 A1 discloses sterile packaging for medical devices.

### SUMMARY

In certain aspects, the present invention provides unique medical graft materials. These graft material can define internal chambers or other voids within the graft material which can be used to receive liquid additives, potentially combined with thru-holes in the graft material which can for example serve to pass biological fluids from one side of the graft material to the other after administration of the graft material to a patient.

In additional aspects, the invention provides advantageous arrangements of graft materials with other device or apparatus features such as liquid input ports or passages, trays, handle structures, and./or other features. In certain embodiments, a tray enclosure is provided that compresses the graft material in selected regions. This compression pattern can serve to facilitate defining a path of fluid flow or collection within the graft material. Additionally or alternatively, the tray enclosure can define a liquid capture well or drain region, to capture fluid that passes through a liquid permeable graft material, for example depositing cells or other bioactive materials on the graft material during that passage through the graft material.

Still additional features and embodiments of the invention as well as advantages associated therewith will be apparent to those of ordinary skill in the art from the descriptions herein, including the appended claims.

### BRIEF DESCRIPTION OF THE FIGURES

FIG. 1 provides a top view of a graft material according to one embodiment of the invention.
FIG. 2 provides a cross-sectional view taken along line 2-2 of FIG. 1 and viewed in the direction of the arrows.
FIG. 3 provides a top view of the graft material embodiment of FIG. 1 in combination with a removable handle structure also defining a receiver, and a liquid input device for receipt in the receiver.
FIG. 4 provides a top end view of the handle structure/graft material combination of FIG. 3.
FIG. 5 provides a bottom end view of the handle structure/graft material combination of FIG. 3.
FIG. 6 provides a top view of the internal features of first and second mating tray portions configured to receive the handle structure/graft material combination of FIGs. 3 to 5.
FIG. 7 provides a top view of one of the tray portions of FIG. 6 having received thereon the handle structure/graft material combination of FIGs. 3 to 5.
FIG. 8 provides a perspective view of the assembled graft material apparatus of the components illustrated in FIGs. 1 to 7, in combination with a syringe coupled to the liquid input device of FIG. 3.
FIG. 9 illustrates another embodiment of a medical graft apparatus.

### DETAILED DESCRIPTION

For the purposes of promoting an understanding of the principles of the invention, reference will now be made to the embodiments illustrated in the drawings and specific language will be used to describe the same. It will nevertheless be understood that no limitation of the scope of the invention is thereby intended, such alterations and further modifications in the illustrated device, and such further applications of the principles of the invention as illustrated therein being contemplated as would normally occur to one skilled in the art to which the invention relates.

As disclosed above, the present invention provides materials and devices that are useful as medical grafts, apparatuses including the same, and related methods of preparation.

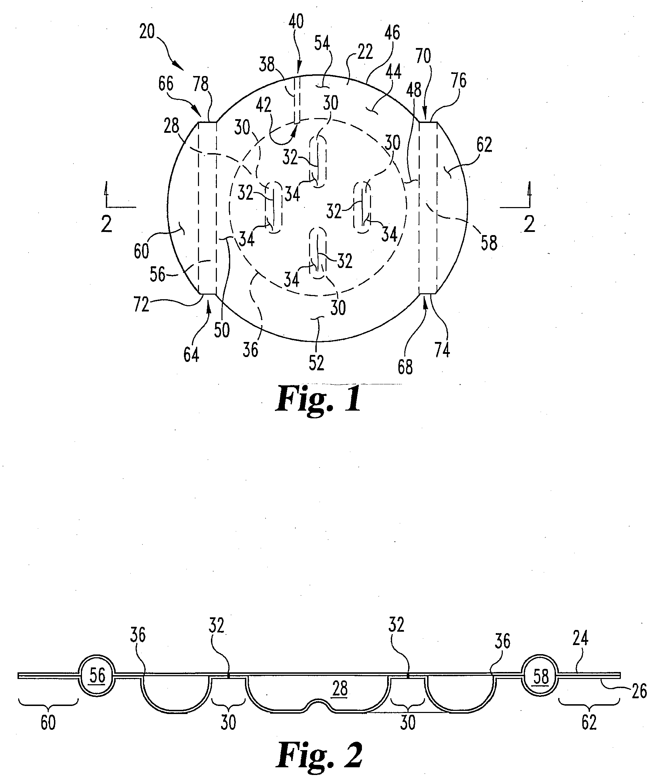

Referring now to Figs. **1** through **8****,** illustrative embodiments will be described. It will be understood that the invention is not limited to these illustrative embodiments; rather a wide variety of additional designs for the disclosed graft materials and devices will be readily envisioned and practicable by those skilled in the art from the disclosure herein.

Generally, shown in **Figs. 1** and **2** are a top view and a cross sectional view, respectively, of a modifiable medical graft **20.** Medical graft **20** includes a graft material body **22** with several unique features. Graft body **22** in the illustrative embodiment is a laminate structure including a first sheet material **24** laminated to a second sheet material **26** (**Fig. 2**). The sheets **24** and **26** are joined to one another in selective areas and left unjoined in other areas. In a first unjoined region the sheets **24** and **26** define an interior chamber **28.** Interior chamber **28** is interrupted by at least one and preferably a plurality of regions **30** in which sheet **24** is laminated to sheet **26** within the confines of the outer perimeter of chamber **28,** thereby dividing chamber **28** into a plurality of chamber regions that are fluidly coupled to one another within the confines of the overall chamber **28.** Joined regions **30** of graft material are thereby fluidly isolated from the volume defined by compartment **28.** Preferably, within joined regions **30** there are provided thru-holes **32** such as slits, perforations or other openings, which define passages through both sheets **24** and **26** in the laminated regions **30.** In this manner, thru-holes **32** can allow for the passage of fluids, such as bodily liquids, from one side of graft body **20** to another opposite side of graft body **20.** At the same time, because joined regions **30** fluidly isolate the thru-holes **32** from the interior volume of chamber **28,** the presence of thru-holes **32** does not cause leakage of fluids received in chamber **28** from the graft body **20.** In the illustrated embodiment this is accomplished by providing a peripheral region **34** of the joined region **30** that surrounds the thru-hole **32** and isolates the thru-hole **32** from the interior chamber **28.** In these peripheral regions **34,** the sheets **24** and **26** are sufficiently laminated to one another to fluidly isolate thru-holes **32** from the volume of chamber **28.**

Chamber **28** has an outer periphery **36** defined at the boundary between joined and unjoined regions of sheets **24** and **26.** In the illustrated embodiment, this periphery is shown as being substantially circular although it will be understood that other shapes such as polygonal or curved shapes may also be used. Chamber **28** and other chambers in grafts herein may be formed in any suitable fashion, e.g. by molds that selectively compress on areas of stacked ECM or other sheets to bond the sheets to one another (e.g. by dehydrothermal bonding during drying by lyophilization or otherwise, crosslinking with chemical or other agents, bonding agents, or other bonding techniques) in those compressed areas while leaving an internal region of the sheets unlaminated to form a chamber, by casting material to form a chamber, or otherwise. During formation of the chamber in the graft material, the chamber may be filled with a substance that is removable after formation of the chamber. This substance may for example be a frozen volume of a liquid such as water that can be removed for example by sublimation during lyophilization, a packing material such as Teflon sheet(s) or strip(s) that can be removed from the chamber after its formation, or another suitable material.

Graft body **20** also includes an open tubular passage **38** extending from an opening **40** at the outer periphery of graft body **20** to an opening **42** opening into chamber **28.** This passage **38** can be used for example to pass fluids into chamber **28** and/or to receive a cannula for passing fluids into chamber **28.** Graft body **20** generally includes a peripheral laminated region **44** in which sheets **24** and **26** are joined to one another, which fluidly isolates chamber **28** from the outer periphery **46** of the graft body **20.** Passage **38** interrupts this fluid isolation and thus fluidly communicates between outer opening **40** and inter opening **42.** The laminated periphery **44** includes uninterrupted joined regions of varying width including lateral laminated regions **48** and **50** and upper and lower laminated regions **52** and **54.** Graft body **20** also includes open tubular passages **56** and **58** generally located within laminated periphery **44.** Passage **56** resides between laminated region **50** and an outermost laminated region **60,** and passage **58** resides between laminated region **48** and outermost laminated region **62.** Passage **56** includes a first opening **64** communicating through passage **58** with a second opening **66.** Passage **58** includes a first opening **68** communicating through passage **58** with second opening **70.** Passages **56** and **58** do not communicate with internal chamber **28** but rather are fluidly isolated therefrom. As will be discussed again later, passages **56** and **58** are for receiving elongated extensions of a handle structure which can be useful for support and/or transfer operations for the graft body **20.** The peripheral laminated region **44** may also have a plurality of thru-holes therein, for example similar to thru-holes **32,** e.g. to allow passage of biological fluid through graft body **20** in this region. As illustrated, the outer periphery of graft body **20** is generally curved but is interrupted by generally straight portions at regions **72, 74, 76** and **78** provided at openings **64, 68, 70** and **66** respectively, with regions **72** and **78** generally running parallel to one another and likewise for regions **74** and **76.**

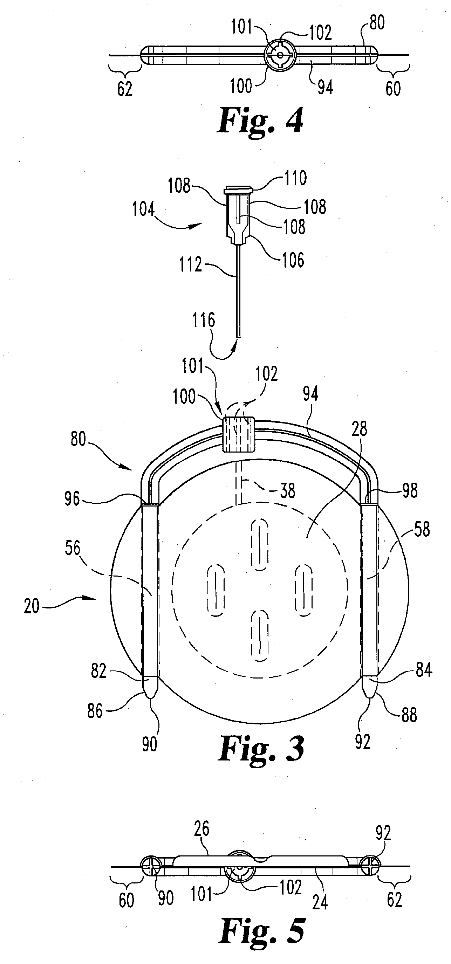

Referring now more particularly to Figs. **3-5** shown is graft body **20** assembled together with a handle structure **80.** Handle structure **80** includes a first prong **82** and second prong **84** which are received in passages **56** and **58** of graft body **20** respectively. First prong **82** includes a tapering region **86** and second prong **84** includes a corresponding tapering region **88,** terminating in generally rounded tips **90** and **92** respectively. Handle structure **80** includes a connecting portion **94** connecting prongs **82** and **84.** As illustrated, connecting portion **94** is a generally smoothly curved region providing handle structure **80** overall as a "U" shaped member. Handle structure **80** also defines a first outwardly extending annular shoulder **96** and a second outwardly extending annular shoulder **98.** Shoulders **96** and **98** have a diameter or other outermost dimension that exceeds the diameter or outermost dimension of passages **56** and **58** and thereby provide stops which resist further travel of prongs **82** and **84** through passages **56** and **58** respectively. Handle structure **80** also includes an attached receiver **100** for receiving a securing a fluid input element **104.** Receiver **100** defines a thru-opening **101** and a plurality of notches **102.** Thru-opening **101** is generally axially aligned with passage **38** and graft body **20.** Fluid input element **104** includes a proximal hub **106** that defines a plurality of raised shoulders **108** which are aligned with and received within notches **102** to secure element **104** within opening **101** in non-rotatable fashion. Hub **106** includes a proximal coupling region **100** which can for example carry threads or other arrangements, e.g. as provided by a luer-lock connector, for coupling to a liquid injection device such as a syringe. Fluid input element **104** also includes an input cannula **112** attached to hub **106** and arranged to receive and transfer fluids from the hub **106** to a distal opening **116.** Cannula **112** is sized for receipt within passage **38** when hub **106** is engaged and secured in opening **101** of receiver **100,** preferably with distal opening **116** residing within chamber **28.**

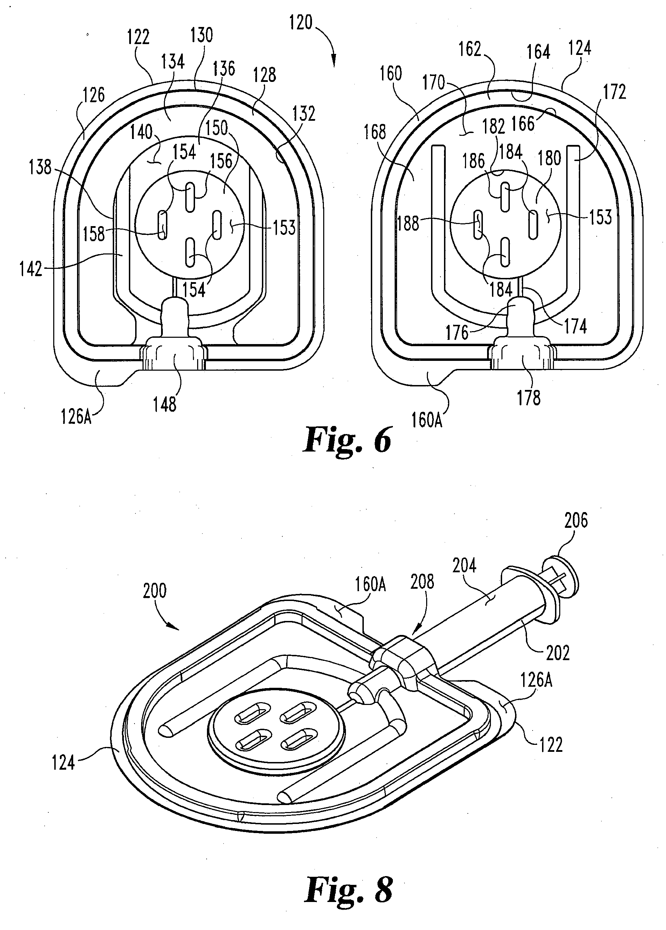

Referring now particularly to Fig. **6****,** shown is a tray **120** that can be used with the assembly provided by handle structure **80,** graft body **20** and fluid input element **104.** Tray **120** includes a lower portion **122** and an upper portion **124.** Lower and upper tray portions **122** and **124** are generally designed to cooperate with one another to receive and support such an assembly. Lower portion **122** includes a peripheral flange **126** and an upstanding shoulder **128** located inward thereof and designed to frictionally mate with a groove **162** defined in upper tray portion **124** as will be discussed later, for example to provide a snap fit. Upstanding shoulder **128** has an outer wall **130** extending upwardly from flange region **126** and an inter wall **132** opposite the outer wall **130.** Positioned inward of shoulder **128** is an internal well **134** which extends around a central region **136** upstanding from well **134** and designed to accommodate the graft body **20**/handle structure **80** assembly discussed above. Upstanding central region **136** includes an outer wall **138** and an upper surface **140.** A generally "U" shaped groove **142** is defined in upper surface **140,** corresponding in shape to the handle structure **80.** Upper surface **136** also has defined therein a groove **144** sized to receive and retain the cannula **112** of fluid input element **104.** Also defined within upper surface **140** is a generally semi-annular depression **146** for accommodating receiver **100** of handle structure **80.** To the outward or peripheral side of depression **146** is another, larger general semi-annular hollow **148** designed to accommodate the distal end of an input device such as a syringe barrel (see e.g. **Fig. 8****,** discussed below).

Surface **140** of central region of **136** also defines a central well sized to receive and accommodate the compartment **29** of graft body **20.** Central well **150** has an outer wall **152** and a lower surface **153.** Upstanding from lower surface **153** are a plurality of pedestals **154** having outer walls **156** and upper surfaces **158.** As can be seen, upper surfaces **158** of pedestals **154** are sized and shaped generally to correspond to the size and shape of the joined regions **32** that divide chamber **28** of graft body **20.**

Upper tray portion **124** includes a peripheral flange **160** which generally corresponds to flange portion **126** of lower tray portion **122.** Inward of flange **160** lies a groove **162** having an outer wall **164** and an inner wall **166.** As discussed above, groove **162** cooperates with shoulder **128** of lower tray portion **122** in the secure, friction-fit closure of the overall tray **120.** For these purposes the outer and inner walls **130** and **132** of tray portion **122** can frictionally engage the outer and inner walls **164** and **166** of upper tray portion **124.** Inward of groove **162** is a raised central region **168** having an upper surface **170.** Upper surface **170** has a generally "U" shaped groove **172** defined herein, which cooperates with groove **142** of lower tray portion **122** to accommodate and support handle structure **80.** A generally semi-annular hollow **176** is also defined from upper surface **170,** which cooperates with hollow **146** of lower tray portion **122** to accommodate and support receiver **100** of handle structure **80.** Peripheral to hollow **176** is a generally larger semi-annular hollow **178,** which cooperates with hollow **148** of lower tray portion **122** to accommodate the distal end region of and end put device such as a syringe, as discussed above.

A central well **180** is defined in upper surface **170,** corresponding in size and shape to central well **150** of lower tray portion **122.** Well **180** includes an outer peripheral wall **182** and a lower surface **183.** Upstanding from lower surface **183** are a plurality of pedestals **184** having outer walls **186** and upper surfaces **188.** As can be seen, pedestals 184 are similar in size and shape to pedestals 154 of lower tray portion 122, and positioned to be in registry therewith when tray portions 122 and 124 are mated together. Pedestals 184 and 154 can contact and compress the graft body 20 on opposite sides thereof when the upper and lower tray portions 122 and 124 are mated together so as to compress joined regions 32 of graft body 20. This can serve to support joined regions 32 during the application of liquids into chamber 28, for instance providing resistance to potential separation (e.g. delamination) of sheets 24 and 26 in joined regions 32. In similar fashion, portions of surface 140 of tray portion 122 can cooperate with portions of surface 170 of tray portion 124 to compress the graft body 20 when the tray portions **122** and **124** are mated together over graft body **20,** including in regions outside and around the periphery **36** of chamber **28,** and in regions adjacent passage **38.** This can again support the graft material in these regions and provide resistance to separation of sheets **24** and **26** during handling or during introduction of liquids into passage **38** and/or chamber **28,** thus maintaining the integrity of the passage **38** and chamber **28.** It will be understood that such compression by a tray enclosure in selective regions can be provided even in the case of graft bodies **20** or portions thereof which are not laminated structures (for example homogenous cast, molded or formed structures), and that the compression can nonetheless help to maintain the structural integrity of the graft material and/or can aid in directing patterns of flow during the application of liquids to the graft material.

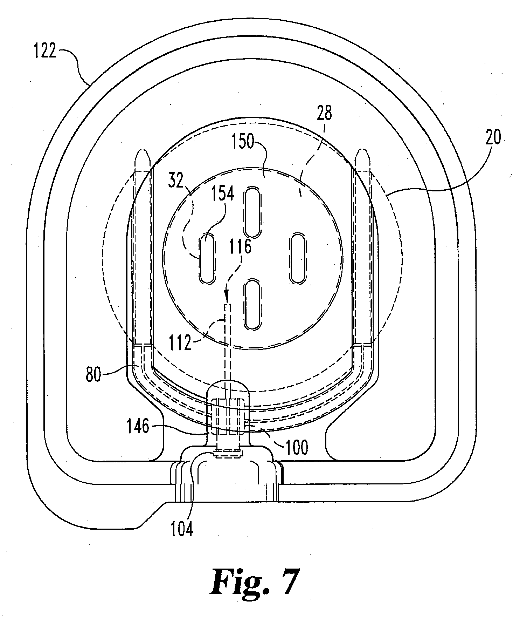

**Fig. 7** shows a view of lower tray portion **122** with the assembly including graft body **20,** handle structure **180** and fluid input element **104** received thereon. As can be seen, the central region **136** receives and accommodates graft body **120** with the chamber **28** of graft body **20** aligned with well **150** of tray portion **122.** As well, handle structure **80** as received and accommodated within groove **142,** receiver **100** is received and accommodated in hollow **146,** and joined regions **32** of graft body **20** are received over and aligned with pedestals **154.** Further, in the assembled structure, fluid input element **104** is received within the opening **101** of receiver **100** with cannula **112** received thru passage **38** of graft body **20** having its distal opening **116** received within chamber **28** of graft body **20.**

With reference to **Fig. 8****,** shown is medical graft system **200** that includes tray portions **122** and **124** assembled to provide tray **120** having modifiable graft **20** received therein (not visible). With the tray **120** so assembled, the tray **120** can compress selected regions of the graft body **20** as discussed above. Additionally, a fluid injection device **202,** such as a syringe as illustrated, is fluidly coupled to fluid element **104** which in turn is received thru passage **38** in graft **20** having its distal opening **116** positioned within chamber **28** of graft **20.** The fluid connection of fluid delivery device **202** with fluid delivery element **104** can be by any suitable means including by a threaded connection between device **202** and threaded proximal end **110** of hub **106,** for example as provided by a luer-lock connection. Device **202** can include a syringe barrel **204** and a plunger **206** received therein and operable to advance within barrel **204** so as to dispel fluids from a distal tip region **208.** In system **200,** upon transfer of fluids from device **202** through fluid delivery element **104** and into chamber **28,** materials such as cells or other suspended particulates, or solutes, can be introduced into chamber **28** and can at least in part deposit upon or within at least portions of graft body **22.** In certain embodiments, the graft material is liquid permeable, and at least a portion of the liquid or other applied fluid can transfer thru the material of graft body **22,** such as sheets **24** and/or **26,** and be drained by gravity or otherwise into the capture well formed by well **150** and well **180.** In addition or alternatively, the material of graft body **22** such as that of sheets **24** and **26** can be flexible or compliant such that the passage of fluids into compartment **28** inflates chamber **28** to a larger dimension. During or after this inflation process, fluids transferred into chamber **28** can at least in part transfer through the graft material, but at a rate slower than their introduction into compartment **28,** such that compartment **28** is inflated. The graft material of graft body **20** can be of sufficiently pliant when wet that when administered to the patient, the upper and lower walls of chamber **28** collapse against one another to flatten the graft body **20,** optionally to essentially a flat sheet graft.

In a further use operation, after the transfer of fluids or materials into chamber **28,** the upper tray portion **124** can be separated from the lower tray portion **122,** for example using tab regions **126A** and/or **160A.** Where liquid has drained into well **150,** lower tray portion **122** may be maintained in a relatively level condition to retain the fluids by gravity within well **150.** The modified graft **20** can be lifted out of lower tray portion **122,** for example using fluid input device **202** (if still connected) and/or using fluid input element or handle structure **80.** After this, the graft **20** can be transferred to a region of the patient in need of graft **20,** for example an ulcer or other topically-exposed wound on the patient. Graft **20** can then be separated from handle structure **80,** with this separation achieved by cutting the graft body **20** and/or by withdrawing the prongs **82** and **84** from passages **56** and **58** of graft body **20.** During or after this operation, if needed, the graft body **20** can be cut to size by the attending health care provider. It will be understood that the fluid input device **202** and/or the fluid input element **104** can be removed from their connection with handle structure **80** at any suitable time during or after a transfer of the graft **20** to the patient.

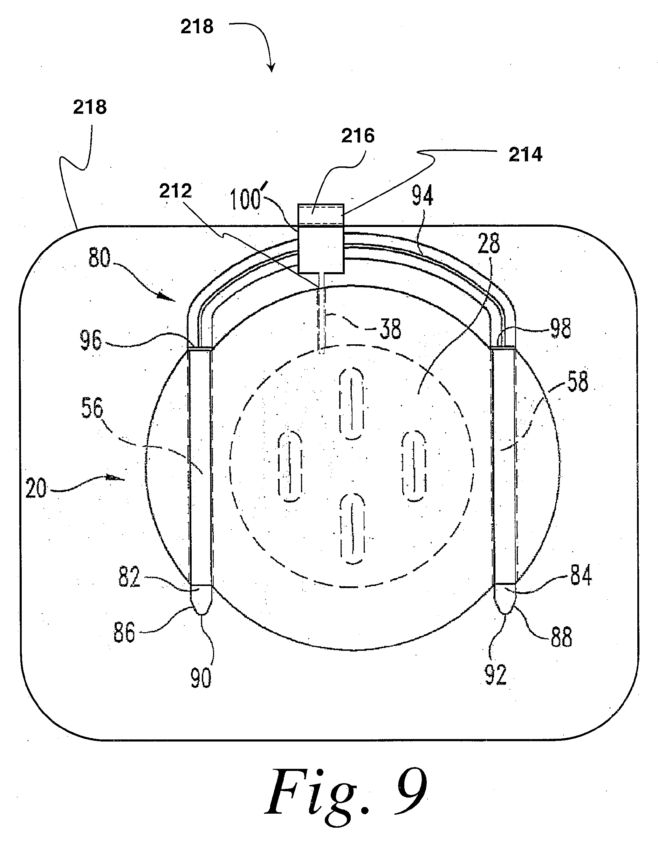

With reference to **Figure 9****,** shown is another embodiment of a medical graft system **210.** Graft system **210** includes a graft assembly as shown in **Figure 3****,** which has components similarly numbered in **Figure 9****.** However, the handle structure **80** includes a modified receiver **100'** which is extended in length relative to receiver **100** of **Figure 3** and provides a tubular passage for delivery of flowable material into the graft body **20.** Receiver **100'** includes a tubular extension **212** with an internal lumen extending through passage **38** of graft body **20** for delivery of liquid to chamber **28.** Receiver **100'** also includes an extended region **214** residing outside of graft body **20** and which houses a needle-penetrable, resealable septum **216.** It will be understood that the overall tubular passage defined by receiver **100** can be a single integral piece or may be multiple connected pieces. System **210** also includes a sterile enclosure **218** such as a polymer film or other package, in which graft body **20** and at least a portion of the combined receiver **100'**/handle **80** structure is sterilely enclosed. It will be understood that in other embodiments the tubular passage-providing receiver and handle may be separate pieces (unattached to one another), in which case at least a portion of the receiver, and potentially only a portion thereof, and at least a portion of the handle, and potentially only a portion or all of the handle, can be sterilely enclosed within the sterile enclosure. Septum **216** can be exposed exterior of the sterile enclosure **218** and when so positioned also provides a sterile seal within the tubular passage of receiver **100'** to maintain sterility within the sterile enclosure **218.** For these purposes the material of the sterile enclosure **218** may be bonded to or otherwise sealed around a periphery of receiver **100'.** The entire system **210** can also be sterilely packaged within a second sterile enclosure such as a polymer film or other package for purposes of storage, handling and/or shipping, if desired. Conventional sterilization processes such as radiation or ethylene oxide sterilization may be used for these purposes. Similar sterile enclosure(s) and sterilization methodology can be used for all embodiments disclosed and encompassed herein.

In use, to deliver a liquid or other flowable additive, such as any of those described herein, into chamber **38** of graft body **20,** a needle with an attached syringe can be used to penetrate septum **216,** and the additive injected into the internal region of the receiver **100'** so as to pass through tubular extension **212** and into chamber. Passage of the needle through the septum **216** and subsequent removal of the needle from the septum **216** maintains the sterility of the environment within sterile enclosure **218.** If desired, and especially in the case of a cellular composition added to the graft body **20,** the resulting graft body **20** can be incubated for a period of time to allow for attachment and/or proliferation of the added cells. In certain modes, the graft body with the cells or other additive is incubated at physiologic temperatures, for example about 37 to about 38 °C, for a period of up to about 12 hours, for example about 1 to about 3 hours.

Whether the system **210** has been incubated or not, when desired, the sterile enclosure **218** can be removed and the graft body **20** can be delivered to a graft site of a patient. This may include use of the handle structure **80** to assist in placing the graft body, as discussed herein, and subsequent removal of the handle structure **80** from the graft body **20.** In this regard, while the handle structure **80** depicted in the preferred, illustrated embodiments is slidably removable from the graft body **20,** other arrangements can be used, including for example a bonding or other permanent attachment of the handle structure to a region (e.g. the periphery) of the graft body, and unneeded portions of the graft body can be cut to separate the handle structure from the graft body portion(s) to remain at the patient site to be treated.

### Graft Materials for Use in Inventive Embodiments

In some embodiments, graft materials of and used in the invention comprise extracellular matrix (ECM) tissue, beneficially in the form of an isolated, decellularized ECM tissue layer. The ECM tissue can be obtained from a warm-blooded vertebrate animal, such as an ovine, bovine or porcine animal. For example, suitable ECM tissue include those comprising submucosa, renal capsule membrane, dermal collagen, dura mater, pericardium, fascia lata, serosa, peritoneum or basement membrane layers, including liver basement membrane. Suitable submucosa materials for these purposes include, for instance, intestinal submucosa including small intestinal submucosa, stomach submucosa, urinary bladder submucosa, and uterine submucosa. ECM tissues comprising submucosa (potentially along with other associated tissues) useful in the present invention can be obtained by harvesting such tissue sources and delaminating the submucosa-containing matrix from smooth muscle layers, mucosal layers, and/or other layers occurring in the tissue source. Porcine tissue sources are preferred sources from which to harvest ECM tissues, including submucosa-containing ECM tissues.

ECM tissue when used in the invention is preferably decellularized and highly purified, for example, as described in U.S. Patent No. 6,206,931 to Cook et al. or U.S. Patent Application Publication No. US2008286268 dated November 20, 2008, publishing U.S. Patent Application Serial No. 12/178,321 filed July 23, 2008. Preferred ECM tissue material will exhibit an endotoxin level of less than about 12 endotoxin units (EU) per gram, more preferably less than about 5 EU per gram, and most preferably less than about 1 EU per gram. As additional preferences, the submucosa or other ECM material may have a bioburden of less than about 1 colony forming units (CFU) per gram, more preferably less than about 0.5 CFU per gram. Fungus levels are desirably similarly low, for example less than about 1 CFU per gram, more preferably less than about 0.5 CFU per gram. Nucleic acid levels are preferably less than about 5 µg/mg, more preferably less than about 2 µg/mg, and virus levels are preferably less than about 50 plaque forming units (PFU) per gram, more preferably less than about 5 PFU per gram. These and additional properties of submucosa or other ECM tissue taught in U.S. Patent No. 6,206,931 or U.S. Patent Application Publication No. US2008286268 may be characteristic of any ECM tissue used in the present invention.

In certain embodiments, the ECM tissue material used as or in the graft material will be a membranous tissue with a sheet structure as isolated from the tissue source. The ECM tissue can, as isolated, have a layer thickness that ranges from about 50 to about 250 microns when fully hydrated, more typically from about 50 to about 200 microns when fully hydrated, although isolated layers having other thicknesses may also be obtained and used. These layer thicknesses may vary with the type and age of the animal used as the tissue source. As well, these layer thicknesses may vary with the source of the tissue obtained from the animal source.

The ECM tissue material utilized desirably retains a structural microarchitecture from the source tissue, including structural fiber proteins such as collagen and/or elastin that are non-randomly oriented. Such non-random collagen and/or other structural protein fibers can in certain embodiments provide an ECM tissue that is non-isotropic in regard to tensile strength, thus having a tensile strength in one direction that differs from the tensile strength in at least one other direction.

The ECM tissue material may include one or more bioactive agents native to the source of the ECM tissue material and retained in the ECM tissue material through processing. For example, a submucosa or other remodelable ECM tissue material may retain one or more native growth factors such as but not limited to basic fibroblast growth factor (FGF-2), transforming growth factor beta (TGF-beta), epidermal growth factor (EGF), cartilage derived growth factor (CDGF), and/or platelet derived growth factor (PDGF). As well, submucosa or other ECM materials when used in the invention may retain other native bioactive agents such as but not limited to proteins, glycoproteins, proteoglycans, and glycosaminoglycans. For example, ECM materials may include heparin, heparin sulfate, hyaluronic acid, fibronectin, cytokines, and the like. Thus, generally speaking, a submucosa or other ECM material may retain from the source tissue one or more bioactive components that induce, directly or indirectly, a cellular response such as a change in cell morphology, proliferation, growth, protein or gene expression.

Submucosa-containing or other ECM materials used in the present invention can be derived from any suitable organ or other tissue source, usually sources containing connective tissues. The ECM materials processed for use in the invention will typically include abundant collagen, most commonly being constituted at least about 80% by weight collagen on a dry weight basis. Such naturally-derived ECM materials will for the most part include collagen fibers that are non-randomly oriented, for instance occurring as generally uniaxial or multiaxial but regularly oriented fibers. When processed to retain native bioactive factors, the ECM material can retain these factors interspersed as solids between, upon and/or within the collagen fibers. Particularly desirable naturally-derived ECM materials for use in the invention will include significant amounts of such interspersed, non-collagenous solids that are readily ascertainable under light microscopic examination with appropriate staining. Such non-collagenous solids can constitute a significant percentage of the dry weight of the ECM material in certain inventive embodiments, for example at least about 1%, at least about 3%, and at least about 5% by weight in various embodiments of the invention.

The submucosa-containing or other ECM material used in the present invention may also exhibit an angiogenic character and thus be effective to induce angiogenesis in a host engrafted with the material. In this regard, angiogenesis is the process through which the body makes new blood vessels to generate increased blood supply to tissues. Thus, angiogenic materials, when contacted with host tissues, promote or encourage the formation of new blood vessels into the materials. Methods for measuring in vivo angiogenesis in response to biomaterial implantation have recently been developed. For example, one such method uses a subcutaneous implant model to determine the angiogenic character of a material. See, C. Heeschen et al., Nature Medicine 7 (2001), No. 7, 833-839. When combined with a fluorescence microangiography technique, this model can provide both quantitative and qualitative measures of angiogenesis into biomaterials. C. Johnson et al., Circulation Research 94 (2004), No. 2, 262-268.

Further, in addition or as an alternative to the inclusion of such native bioactive components, non-native bioactive components such as those synthetically produced by recombinant technology or other methods (e.g., genetic material such as DNA), may be incorporated into an ECM material used in the invention. These non-native bioactive components may be naturally-derived or recombinantly produced proteins that correspond to those natively occurring in an ECM tissue, but perhaps of a different species. These non-native bioactive components may also be drug substances. Illustrative drug substances that may be added to materials include, for example, anti-clotting agents, e.g. heparin, antibiotics, antiinflammatory agents, thrombus-promoting substances such as blood clotting factors, e.g., thrombin, fibrinogen, and the like, and anti-proliferative agents, e.g. taxol derivatives such as paclitaxel. Such non-native bioactive components can be incorporated into and/or onto ECM material in any suitable manner, for example, by surface treatment (e.g., spraying) and/or impregnation (e.g., soaking), just to name a few. Also, these substances may be applied to the ECM material in a premanufacturing step, immediately prior to the procedure (e.g., by soaking the material in a solution containing a suitable antibiotic such as cefazolin), or during or after engraftment of the material in the patient.

Inventive graft compositions herein can incorporate xenograft ECM material (i.e., cross-species material, such as tissue material from a non-human donor to a human recipient), allograft ECM material (i.e., interspecies material, with tissue material from a donor of the same species as the recipient), and/or autograft ECM material (i.e., where the donor and the recipient are the same individual). Further, any exogenous bioactive substances incorporated into an ECM material may be from the same species of animal from which the ECM material was derived (e.g. autologous or allogenic relative to the ECM material) or may be from a different species from the ECM material source (xenogenic relative to the ECM material). In certain embodiments, ECM tissue material will be xenogenic relative to the patient receiving the graft, and any added cells or other exogenous material(s) will be from the same species (e.g. autologous or allogenic) as the patient receiving the graft. Illustratively, human patients may be treated with xenogenic ECM materials (e.g. porcine-, bovine- or ovine-derived) that have been modified with exogenous human cells and/or serum proteins and/or other material(s) as described herein, those exogenous materials being naturally derived and/or recombinantly produced.

When used in the invention, ECM materials can be free or essentially free of additional, non-native crosslinking, or may contain additional crosslinking. Such additional crosslinking may be achieved by photo-crosslinking techniques, by chemical crosslinkers, or by protein crosslinking induced by dehydration or other means. However, because certain crosslinking techniques, certain crosslinking agents, and/or certain degrees of crosslinking can destroy the remodelable properties of a remodelable material, where preservation of remodelable properties is desired, any crosslinking of the remodelable ECM material can be performed to an extent or in a fashion that allows the material to retain at least a portion of its remodelable properties. Chemical crosslinkers that may be used include for example aldehydes such as glutaraldehydes, diimides such as carbodiimides, e.g., 1-ethyl-3-(3-dimethylaminopropyl)carbodiimide hydrochloride, ribose or other sugars, acyl-azide, sulfo-N-hydroxysuccinamide, or polyepoxide compounds, including for example polyglycidyl ethers such as ethyleneglycol diglycidyl ether, available under the trade name DENACOL EX810 from Nagese Chemical Co., Osaka, Japan, and glycerol polyglycerol ether available under the trade name DENACOL EX 313 also from Nagese Chemical Co. Typically, when used, polyglycerol ethers or other polyepoxide compounds will have from 2 to about 10 epoxide groups per molecule.

In additional embodiments, substrates of the invention can be made from ECM's or other collagenous materials that have been subjected to processes that expand the materials. In certain forms, such expanded materials can be formed by the controlled contact of an ECM material with a denaturing agent such as one or more alkaline substances until the material expands, and the isolation of the expanded material. Illustratively, the contacting can be sufficient to expand the ECM material to at least 120% of (i.e. 1.2 times) its original bulk volume, or in some forms to at least about two times its original volume. Thereafter, the expanded material can optionally be isolated from the alkaline medium, e.g. by neutralization and/or rinsing. The collected, expanded material can be used in any suitable manner in the preparation of a substrate. Illustratively, the expanded material can be enriched with bioactive components, comminuted, dried, and/or molded, etc., in the formation of a substrate of a desired shape or configuration. In certain embodiments, a dried substrate formed with the expanded ECM material can be highly compressible and/or expandable.

Treatment of an ECM material with a denaturant, such as an alkaline material, can cause changes in the physical structure of the material that in turn cause it to expand. Such changes may include denaturation of the collagen in the material. In certain embodiments, it is preferred to expand the material to at least about three, at least about four, at least about 5, or at least about 6 or even more times its original bulk volume. It will be apparent to one skilled in the art that the magnitude of the expansion is related to several factors, including for instance the concentration or pH of the alkaline medium, the exposure time of the alkaline medium to the material, and temperature used in the treatment of the material to be expanded, among others. These factors can be varied through routine experimentation to achieve a material having the desired level of expansion, given the disclosures herein.

A collagen fibril is comprised of a quarter-staggered array of tropocollagen molecules. The tropocollagen molecules themselves are formed from three polypeptide chains linked together by covalent intramolecular bonds and hydrogen bonds to form a triple helix. Additionally, covalent intermolecular bonds are formed between different tropocollagen molecules within the collagen fibril. Frequently, multiple collagen fibrils assemble with one another to form collagen fibers. It is believed that the addition of an alkaline substance to the material as described herein can be conducted so as to not significantly disrupt the intramolecular and intermolecular bonds, but denature the material to an extent that provides to the material an increased processed thickness, e.g. at least twice the naturally-occurring thickness. ECM materials that can be processed to make expanded materials for use as substrates can include any of those disclosed herein or other suitable ECM's. Typical such ECM materials will include a network of collagen fibrils having naturally-occurring intramolecular cross links and naturally-occurring intermolecular cross links. Upon expansion processing as described herein, the naturally-occurring intramolecular cross links and naturally-occurring intermolecular cross links can be retained in the processed collagenous matrix material sufficiently to maintain the collagenous matrix material as an intact collagenous sheet material; however, collagen fibrils in the collagenous sheet material can be denatured, and the collagenous sheet material can have an alkaline-processed thickness that is greater than the thickness of the starting material, for example at least 120% of the original thickness, or at least twice the original thickness. The expanded ECM material can then be processed to provide foam or sponge substrates, e.g. by comminuting, casting, and drying the processed material. Additional information concerning expanded ECM materials and their preparation is found in United States Patent Application Publication No. US20090326577 published December 31, 2009, publishing United States Patent Application Serial No. 12/489,199 filed June 22, 2009.

In addition to or as an alternative to ECM materials, the graft material used in the invention may be comprised of other suitable materials. Illustrative materials include, for example, synthetically-produced substrates comprised or natural or synthetic polymers. Illustrative synthetic polymers can include nonresorbable synthetic biocompatible polymers, such as cellulose acetate, cellulose nitrate, silicone, polyethylene teraphthalate, polyurethane, polyamide, polyester, polyorthoester, polyanhydride, polyether sulfone, polycarbonate, polypropylene, high molecular weight polyethylene, polytetrafluoroethylene, or mixtures or copolymers thereof; or resorbable synthetic polymer materials such as polylactic acid, polyglycolic acid or copolymers thereof, polyanhydride, polycaprolactone, polyhydroxy-butyrate valerate, polyhydroxyalkanoate, or another biodegradable polymer or mixture thereof. Preferred graft materials comprised of these or other materials will be porous matrix materials configured to allow cellular invasion and ingrowth into the matrix.

Extracellular matrix tissue layers can be used in the manufacture of laminated graft body structures, such as that illustrated in graft body **20** illustrative above. For these purposes, each of sheet **24** and **26** can for example be comprised of about 1 to about 10 extracellular matrix tissue layers. Illustratively, either of sheet **24** and **26** can include only a single extracellular matrix tissue layer, and the other may include multiple (e.g. 1 to 10, or 2 to 6) extracellular matrix tissue layers. Sheets of multilaminate ECM tissue layers can be prepared in any suitable fashion. These include, for instance, dehydrothermal bonding under heated, non-heated or lyophilization conditions, using adhesives, glues or other bonding agents, crosslinking with chemical agents or radiation (including UV radiation), or any combination of these with each other or other suitable methods. For additional information as to techniques for laminating ECM layers to one another, reference may be made for example to U.S. Patent Nos. 5,711,969, 5,755,791, 5,855,619, 5,955,110, 5,968,096, and to U.S. Patent Publication No. 20050049638. Further, where sheets **24** and **26** are laminated to one another, these same techniques may for example be used. The lamination of layers within sheets **24** and **26** and the lamination of selected regions of sheets **24** and **26** to one another can also be accomplished simultaneously using these or other suitable procedures.

In certain embodiments, sheets **24** and **26** are laminated in selected regions, e.g. as discussed above for graft body **20,** using a preparative procedure with a two-piece mold. In such a procedure, sheets **24** and **26** can be provided in a wetted condition. Sheet **26** can be laid upon and pressed into a first mold piece having a surface contour which is the same or approximately the same as the graft-accommodating region of tray portion **122.** Thus, sheet **26** can be tucked into and around a well and pedestals of the first mold piece that match the well **150** and pedestals **154** of tray portion **122,** and into a "U" shaped groove that matches groove **142** of tray portion **122.** The handle structure **80** and the cannula **112,** pieces having matching structures and dimensions, can then be laid onto sheet **26.** Sheet **24** can then be laid onto this assembly, and for example pulled fairly taught (e.g. to result in the flat upper sheet **24** as depicted in graft body **20,** along with the tufted lower sheet **26.** A second mold piece having a surface contour including grooves that match groove **172** and groove **174** of tray portion **124,** and which compresses other areas of the sheets **24** and **26** to be joined, is then clamped over the first mold piece. The first and second mold pieces, when so clamped, compress the sheets **24** and **26** against one another where they are to be laminated together, and do not compress the sheets **24** and 26 in other areas. The clamped mold assembly can then be dried, preferably by lyophilization. This can be used to provide a dried graft body **20** in which the ECM tissue in the laminated areas is less porous (and/or more dense) than the ECM tissue in the non-laminated areas. If desired, after clamping the mold over the wetted sheets **24** and **26** and prior to drying, a liquid such as water can be injected into the chamber **28** to inflate and assure separation of the walls defining it, to reduce the risk of unintended lamination of these walls to one other in the region intended for chamber **28** during the drying process. The mold can be provided with an injection port and drain holes to facilitate these processes. After drying the assembly can be deconstructed to obtain the graft body **20** or a precursor that can be trimmed of excess material to provide the shape of graft body **20.**

### Cells and/or Other Therapeutic Substances For Use in Inventive Embodiments

Any one or any combination of a wide variety of cell types can be applied to graft materials as disclosed herein to provide cellular graft compositions and methods of the invention. For example, the cells can be skin cells, skeletal muscle cells, cardiac muscle cells, lung cells, mesentery cells, or adipose cells. The adipose cells may be from omental fat, properitoneal fat, perirenal fat, pericardial fat, subcutaneous fat, breast fat, or epididymal fat. In certain embodiments, the cells comprise stromal cells, stem cells, or combinations thereof. As used herein, the term "stem cells" is used in a broad sense and includes traditional stem cells, adipose derived stem cells, progenitor cells, preprogenitor cells, reserve cells, and the like. Exemplary stem cells include embryonic stem cells, adult stem cells, pluripotent stem cells, neural stem cells, liver stem cells, muscle stem cells, muscle precursor stem cells, endothelial progenitor cells, bone marrow stem cells, chondrogenic stem cells, lymphoid stem cells, mesenchymal stem cells, hematopoietic stem cells, central nervous system stem cells, peripheral nervous system stem cells, and the like. Additional illustrative cells which can be used include hepatocytes, epithelial cells, Kupffer cells, fibroblasts, neurons, cardiomyocytes, myocytes, chondrocytes, pancreatic acinar cells, islets of Langerhans, osteocytes, myoblasts, satellite cells, endothelial cells, adipocytes, preadipocytes, biliary epithelial cells, and progentior cells of any of these cell types.

In some embodiments, the cells incorporated in the cellular grafts are, or include, endothelial progenitor cells (EPCs). Preferred EPCs for use in the invention are endothelial colony forming cells (ECFCs), especially ECFCs with high proliferative potential. Suitable such cells are described for example in U.S. Patent Application Publication No. 20050266556 published December 1, 2005, publishing U.S. Patent Application Serial No. 11/055,182 filed February 9, 2005, and U.S. Patent Application Publication No. 20080025956 published January 1, 2008, publishing U.S. Patent Application No. 11/837,999, filed August 13, 2007. Such ECFC cells can be a clonal population, and/or can be obtained from umbilical cord blood of humans or other animals. Additionally or alternatively, the endothelial colony forming cells have the following characteristics: (a) express the cell surface antigens CD31, CD105, CD146, and CD144; and/or (b) do not express CD45 and CD 14; and/or (c) ingest acetylated LDL; and/or (d) replate into at least secondary colonies of at least 2000 cells when plated from a single cell; and/or (e) express high levels of telomerase, at least 34% of that expressed by HeLa cells; and/or (f) exhibit a nuclear to cytoplasmic ratio that is greater than 0.8; and/or (g) have cell diameters of less than about 22 microns. Any combination of some or all of these features (a)-(g) may characterize ECFCs used in the present invention.

In other embodiments, the cells incorporated in the cellular grafts are, or include, muscle derived cells, including muscle derived myoblasts and/or muscle derived stem cells. Suitable such stem cells and methods for obtaining them are described, for example, in U.S. Patent No. 6,866,842 and U.S. Patent No. 7,155,417, each of which is hereby incorporated herein by reference in its entirety. The muscle derived cells can express desmin, M-cadherin, MyoD, myogenin, CD34, and/or Bcl-2, and can lack expression of CD45 or c-Kit cell markers.

In still other embodiments, the cells incorporated in the cellular grafts are, or include, stem cells derived from adipose tissue. Suitable such cells and methods for obtaining them are described for example in U.S. Patent No. 6,777,231 and U.S. Patent No. 7,595,043. The cellular population can include adipose-derived stem and regenerative cells, sometimes also referred to as stromal vascular fraction cells, which can be a mixed population including stem cells, endothelial progenitor cells, leukocytes, endothelial cells, and vascular smooth muscle cells, which can be adult-derived. In certain forms, cellular grafts of the present invention can be prepared with and can include adipose-derived cells that can differentiate into two or more of a bone cell, a cartilage cell, a nerve cell, or a muscle cell.
In addition to or as an alternative to cells, a liquid or otherwise flowable additive to be added to the graft can include other therapeutic substances. These substances may for example include growth factors, pharmaceutical agents, biologic materials or extracts such as platelets or platelet rich plasma, platelet lysates, blood or bone marrow fractions, and/or extracellular matrix particles or gels that optionally retain biologically active substances native to their source tissue such as growth factors, glycosaminoglycans, and/or proteoglycans. These and still other therapeutic substances known to those skilled in the art can be delivered into an internal chamber of the graft e.g. as described herein.

### Medical Treatments with Grafts

(the medical treatments not being part of the invention)

Grafts of and prepared in accordance with the invention can be used in a wide variety of clinical applications to treat damaged, diseased or insufficient tissues, and can be used in humans or in non-human animals. Such tissues to be treated may, for example, be muscle tissue, nerve tissue, brain tissue, blood, myocardial tissue, cartilage tissue, organ tissue such as lung, kidney or liver tissue, bone tissue, arterial or venous vessel tissue, skin tissue, and others.

In certain embodiments, the grafts can be used to enhance the formation of blood vessels in a patient, for example to alleviate ischemia in tissues. Direct administration of blood vessel-forming cellular grafts, for example grafts containing endothelial colony forming cells or other endothelial progenitor cells, to an ischemic site can enhance the formation of new vessels in the affected areas and improve blood flow or other outcomes. The ischemic tissue to be treated may for example be ischemic myocardial tissue, e.g. following an infarction, or ischemic tissue in the legs or other limbs such as occurs in critical limb ischemia.

The grafts of the invention can also be used to enhance the healing of partial or full thickness dermal wounds, such as skin ulcers, e.g. diabetic ulcers, and burns. Illustratively, the administration of grafts containing cells, for example endothelial colony forming cells or other endothelial progenitor cells, to such wounds can enhance the healing of the wounds.

In other applications, the grafts can be used to generate muscle tissue at a target site, for example in the treatment of skeletal muscle tissue, smooth muscle tissue, myocardial tissue, or other tissue. Illustratively, grafts of the invention containing muscle derived myoblasts can be implanted into muscle tissue of a sphincter such as a urinary bladder sphincter to treat incontinence.

The uses of the terms "a" and "an" and "the" and similar references in the context of describing the invention (especially in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

While the invention has been illustrated and described in detail in the drawings and foregoing description, the same is to be considered as illustrative and not restrictive in character, it being understood that only the preferred embodiment has been shown and described.

## Claims

1. A medical graft (20), comprising:
a graft material having a first outer surface and a second outer surface, the graft material defining an internal chamber (28) between the first outer surface and the second outer surface of the graft material; and
at least one region (30) of the graft material interrupting the chamber and surrounded by the chamber.

2. The medical graft of claim 1, also comprising a through-hole (32) defined in said at least one region (30) of the graft material, the through-hole extending between the first outer surface and the second outer surface of the graft material.

3. The medical graft of claim 1 or 2, wherein the graft material comprises a matrix for supporting cell growth.

4. The medical graft of any preceding claim, comprising:
a first wall of the graft material defining a first boundary of the chamber (28);
a second wall of the graft material defining a second boundary of the chamber; and
the first and second walls laminated to one another to provide said at least one region of the graft material.

5. The medical graft of claim 4, wherein the first wall and second wall comprise an extracellular matrix material.

6. The medical graft of any preceding claim, comprising a plurality of said regions (30).

7. The medical graft of claim 6, wherein each of said regions (30) defines a through-hole (32).

8. The medical graft of any preceding claim, also comprising a peripheral flange defined by the graft material, the peripheral flange extending around said internal chamber (28).

9. The medical graft of claim 8, also comprising at least one open passage (38) defined by the graft material of the peripheral flange, the open passage extending through the flange and opening into the internal chamber.

10. A medical graft according to any one of claims 1 to 9, also comprising:
a tray enclosure (120) in which the graft material is received.

11. The medical graft of claim 10, wherein the tray enclosure (120) compresses the graft material in selected regions.

12. A medical graft according to claim 10 or 11, wherein the tray enclosure (120) compresses the graft material peripherally around said internal chamber (28) but not on said internal chamber.

13. A medical graft according to any one of claims 1 to 12, also comprising:
a liquid additive applied to the graft material.

14. The medical graft of claim 13, wherein the liquid additive includes cells.

15. A method for preparing a medical graft material, comprising:
delivering liquid additive to the graft material of a medical graft of any of claims 1 to 12.

16. The method of claim 15 wherein said delivering comprises delivering the liquid additive to the internal chamber (28).

17. The method of claim 15 or 16, wherein the liquid additive comprises a therapeutic substance.

18. The method of any one of claims 15 to 17, wherein the liquid additive comprises cells.

## Patentansprüche

1. Medizinisches Transplantat (20), umfassend:
ein Transplantatmaterial mit einer ersten Außenoberfläche und einer zweiten Außenoberfläche, wobei das Transplantatmaterial eine innere Kammer (28) zwischen der ersten Außenoberfläche und der zweiten Außenoberfläche des Transplantatmaterials definiert; und
wenigstens einen Bereich (30) des Transplantatmaterials, der die Kammer unterbricht und von der Kammer umgeben ist.

2. Medizinisches Transplantat gemäß Anspruch 1, ferner umfassend ein Durchgangsloch (32), das in dem wenigstens einen Bereich (30) des Transplantatmaterials definiert ist, wobei das Durchgangsloch zwischen der ersten Außenoberfläche und der zweiten Außenoberfläche des Transplantatmaterials verläuft.

3. Medizinisches Transplantat gemäß Anspruch 1 oder 2, wobei das Transplantatmaterial eine Matrix zum Fördern von Zellwachstum umfasst.

4. Medizinisches Transplantat gemäß einem der vorstehenden Ansprüche, umfassend:
eine erste Wand des Transplantatmaterials, die eine erste Begrenzung der Kammer (28) definiert;
eine zweite Wand des Transplantatmaterials, die eine zweite Begrenzung der Kammer definiert; und
wobei die erste und die zweite Wand aneinanderlaminiert sind, um den wenigstens einen Bereich des Transplantatmaterials bereitzustellen.

5. Medizinisches Transplantat gemäß Anspruch 4, wobei die erste Wand und die zweite Wand ein extrazelluläres Matrixmaterial umfassen.

6. Medizinisches Transplantat gemäß einem der vorstehenden Ansprüche, umfassend eine Vielzahl der Bereiche (30).

7. Medizinisches Transplantat gemäß Anspruch 6, wobei jeder der Bereiche (30) ein Durchgangsloch (32) definiert.

8. Medizinisches Transplantat gemäß einem der vorstehenden Ansprüche, ferner umfassend einen von dem Transplantatmaterial definierten Umfangsflansch, wobei der Umfangsflansch um die innere Kammer (28) verläuft.

9. Medizinisches Transplantat gemäß Anspruch 8, ferner umfassend wenigstens einen von dem Transplantatmaterial des Umfangsflanschs definierten offenen Durchgang (38), wobei der offene Durchgang durch den Flansch verläuft und sich in die innere Kammer öffnet.

10. Medizinisches Transplantat gemäß einem der Ansprüche 1 bis 9, ferner umfassend:
einen Schalenbehälter (120), in den das Transplantatmaterial aufgenommen wird.

11. Medizinisches Transplantat gemäß Anspruch 10, wobei der Schalenbehälter (120) das Transplantatmaterial in ausgewählten Bereichen komprimiert.

12. Medizinisches Transplantat gemäß Anspruch 10 oder 11, wobei der Schalenbehälter (120) das Transplantatmaterial am Umfang um die innere Kammer (28) aber nicht an der inneren Kammer komprimiert.

13. Medizinisches Transplantat gemäß einem der Ansprüche 1 bis 12, ferner umfassend:
einen flüssigen Zusatzstoff, der auf das Transplantatmaterial aufgebracht ist.

14. Medizinisches Transplantat gemäß Anspruch 13, wobei der flüssige Zusatzstoff Zellen enthält.

15. Verfahren zum Herstellen eines medizinischen Transplantatmaterials, umfassend:
Zugeben von flüssigem Zusatzstoff zu dem Transplantatmaterial eines medizinischen Transplantats gemäß einem der Ansprüche 1 bis 12.

16. Verfahren gemäß Anspruch 15, wobei das Zugeben das Zugeben des flüssigen Zusatzstoffs zu der inneren Kammer (28) umfasst.

17. Verfahren gemäß Anspruch 15 oder 16, wobei der flüssige Zusatzstoff einen therapeutischen Stoff umfasst.

18. Verfahren gemäß einem der Ansprüche 15 bis 17, wobei der flüssige Zusatzstoff Zellen umfasst.

## Revendications

1. Greffon médical (20) comprenant :
une matière de greffe comportant une première surface extérieure et une seconde surface extérieure, la matière de greffe délimitant une chambre interne (28) entre la première surface extérieure et la seconde surface extérieure de la matière de greffe ; et
au moins une zone (30) de la matière de greffe interrompant la chambre et entourée par elle.

2. Greffon médical selon la revendication 1, comprenant en outre un trou traversant (32) pratiqué dans ladite zone (30) de la matière de greffe, le trou traversant s'étendant entre la première surface extérieure et la seconde surface extérieure de la matière de greffe.

3. Greffon médical selon la revendication 1 ou 2, dans lequel la matière de greffe comprend une matrice pour soutenir une croissance de cellules.

4. Greffon médical selon l'une quelconque des revendications précédentes, comprenant :
une première paroi de la matière de greffe définissant une première limite de la chambre (28) ;
une seconde paroi de la matière de greffe définissant une seconde limite de la chambre ; et
les première et seconde parois étant laminées l'une à l'autre pour produire ladite zone de la matière de greffe.

5. Greffon médical selon la revendication 4, dans lequel les première et seconde parois comprennent une matière de matrice extracellulaire.

6. Greffon médical selon l'une quelconque des revendications précédentes, comprenant une pluralité desdites zones (30).

7. Greffon médical selon la revendication 6, dans lequel chacune desdites zones (30) délimite un trou traversant (32).

8. Greffon médical selon l'une quelconque des revendications précédentes, comprenant en outre un rebord périphérique défini par la matière de greffe, le rebord périphérique s'étendant autour de ladite chambre interne (28).

9. Greffon médical selon la revendication 8, comprenant en outre au moins un passage ouvert (38) délimité par la matière de greffe du rebord périphérique, le passage ouvert traversant le rebord et débouchant dans la chambre interne.

10. Greffon médical selon l'une quelconque des revendications 1 à 9, comprenant en outre une enceinte (120) de plateau qui reçoit la matière de greffe.

11. Greffon médical selon la revendication 10, dans lequel l'enceinte (120) de plateau comprime la matière de greffe dans des zones choisies.

12. Greffon médical selon la revendication 10 ou 11, dans lequel l'enceinte (120) de plateau comprime la matière de greffe en périphérie autour de ladite chambre interne (28), mais pas sur ladite chambre interne.

13. Greffon médical selon l'une quelconque des revendications 1 à 12, comprenant en outre un additif liquide appliqué sur la matière de greffe.

14. Greffon médical selon la revendication 13, dans lequel l'additif liquide contient des cellules.

15. Procédé de préparation d'une matière de greffon médical comprenant l'opération consistant à distribuer un additif liquide à la matière de greffe d'un greffon médical selon l'une quelconque des revendications 1 à 12.

16. Procédé selon la revendication 15, dans lequel ladite distribution inclut de distribuer l'additif liquide à la chambre interne (28).

17. Procédé selon la revendication 15 ou 16, dans lequel l'additif liquide comprend une substance thérapeutique.

18. Procédé selon l'une quelconque des revendications 15 à 17, dans lequel l'additif liquide contient des cellules.
